(19) **Europäisches Patentamt / European Patent Office / Office européen des brevets**

(11) **EP 2 805 676 A1**

(12) **EUROPEAN PATENT APPLICATION**
published in accordance with Art. 153(4) EPC

(43) Date of publication:
**26.11.2014 Bulletin 2014/48**

(21) Application number: **12866220.2**

(22) Date of filing: **18.01.2012**

(51) Int Cl.:
**A61B 8/00** (2006.01)

(86) International application number:
**PCT/JP2012/050914**

(87) International publication number:
**WO 2013/108375 (25.07.2013 Gazette 2013/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Canon Kabushiki Kaisha Tokyo 146-8501 (JP)**

(72) Inventor: **MIYASATO, Takuro Tokyo 146-8501 (JP)**

(74) Representative: **Derham, Cassandra Virginie et al Canon Europe Ltd European Patent Department 3 The Square Stockley Park Uxbridge, Middlesex UB11 1ET (GB)**

(54) **SUBJECT INFORMATION ACQUISITION DEVICE AND SUBJECT INFORMATION ACQUISITION METHOD**

(57)     In photoacoustic imaging, a subject information obtaining apparatus and a method for obtaining information regarding a subject that can accurately obtain an optical characteristic value are provided.

A subject information obtaining apparatus according to the present invention includes a signal processing unit that includes a setting unit that sets certain sensitive regions corresponding to a plurality of acoustic wave detection elements on the basis of sensitivity distribution of the plurality of acoustic wave detection elements, an initial sound pressure obtaining unit that obtains initial sound pressure in a region of interest without using a detection signal corresponding to the region of interest obtained by an acoustic wave detection element whose certain sensitive region does not include the region of interest, a light intensity obtaining unit that obtains a value of integrated light intensity in the region of interest on the basis of detection signals used by the initial sound pressure obtaining unit, and an optical characteristic value obtaining unit that obtains an optical characteristic value in the region of interest using the initial sound pressure obtained by the initial sound pressure obtaining unit and the value of the integrated light intensity obtained by the light intensity obtaining unit.

FIG. 1

**Description**

Technical Field

The present invention relates to a subject information obtaining apparatus and a method for obtaining information regarding a subject that obtain information regarding a subject by detecting a photoacoustic wave generated by radiating light onto the subject.

Background Art

Studies on an optical imaging apparatus that transmits light through a subject, the light being radiated from a light source such as a laser onto the subject, and that obtains information regarding an inside of the subject are eagerly conducted mainly in the medical field. Photoacoustic imaging (PAI) is one of such optical imaging technologies. The photoacoustic imaging is a technology in which pulse light generated from a light source is radiated onto a subject (living body), a photoacoustic wave generated when the light that has propagated through and diffused in the subject is absorbed in the subject is detected, and the detected acoustic wave is subjected to an analysis process in order to visualize information relating to the optical characteristics of the inside of the subject. By this technology, the distribution of optical characteristic values inside the subject, especially the absorption coefficient distribution, the oxygen saturation distribution, and the like, can be obtained.

In the photoacoustic imaging, initial sound pressure $P_0$ of a photoacoustic wave generated from a region of interest inside the subject can be represented by the following expression.

[Math. 1]

$$P_0 = \Gamma \cdot \mu_\alpha \cdot \Phi \;\; \ldots \; \texttt{Expression (1)}$$

Here, $\Gamma$ is a Grueneisen coefficient, which is obtained by dividing a product of a volume expansion coefficient $\beta$ and the square of a sonic speed c by specific heat at constant pressure $C_P$. It is known that once a subject has been determined, $\Gamma$ indicates a substantially constant value. In addition, $\mu_a$ is an absorption coefficient of the region of interest, and $\Phi$ is a value of the integrated light intensity in the region of interest.

In PTL 1, a technology is described in which changes over time in sound pressure P of a photoacoustic wave that has propagated through a subject are detected by an acoustic wave detector and the distribution of initial sound pressure inside the subject is calculated on the basis of results of the detection. According to PTL 1, by dividing the calculated initial sound pressure by the Grueneisen coefficient $\Gamma$, a product of $\mu_a$ and $\Phi$, that is, light energy absorption density, can be obtained. In addition, as indicated by the expression (1), the light energy absorption density needs to be divided by the light intensity $\Phi$ in order to obtain the absorption coefficient $\mu_a$ from the initial sound pressure $P_0$.

Citation List

Patent Literature

PTL 1: Japanese Patent Laid-Open No. 2010-88627
PTL 2: Japanese Patent Laid-Open No. 2006-51355

Summary of Invention

Technical Problem

However, in the photoacoustic imaging described in PTL 1, it has been desired to obtain an optical characteristic value more accurately.

Therefore, an object of the present invention is to provide, in photoacoustic imaging, a subject information obtaining apparatus and a method for obtaining information regarding a subject that can obtain an optical characteristic value more accurately.

Solution to Problem

In view of the above problem, a subject information obtaining apparatus according to the present invention includes a signal processing unit that includes a setting unit that sets certain sensitive regions corresponding to a plurality of acoustic wave detection elements on the basis of sensitivity distribution of the plurality of acoustic wave detection elements, an initial sound pressure obtaining unit that obtains initial sound pressure in a region of interest without using a detection signal corresponding to the region of interest obtained by an acoustic wave detection element whose certain sensitive region does not include the region of interest, a light intensity obtaining unit that obtains a value of integrated light intensity in the region of interest on the basis of detection signals used by the initial sound pressure obtaining unit, and an optical characteristic value obtaining unit that obtains an optical characteristic value in the region of interest using the initial sound pressure obtained by the initial sound pressure obtaining unit and the value of the integrated light intensity obtained by the light intensity obtaining unit.

Advantageous Effects of Invention

According to the present invention, a subject information obtaining apparatus and a method for obtaining information regarding a subject that can obtain an optical characteristic value more accurately can be provided.

Brief Description of Drawings

[Fig. 1] Fig. 1 is a schematic diagram of a subject information obtaining apparatus according to a first embodiment.
[Fig. 2] Fig. 2 is a flowchart diagram of a method for obtaining information regarding a subject according to the first embodiment.
[Fig. 3A] Fig. 3A is a schematic diagram of a subject information obtaining apparatus according to a second embodiment.
[Fig. 3B] Fig. 3B is a schematic diagram of the subject information obtaining apparatus according to the second embodiment.
[Fig. 3C] Fig. 3C is a schematic diagram of the subject information obtaining apparatus according to the second embodiment.
[Fig. 4A] Fig. 4A is a schematic diagram of a subject information obtaining apparatus according to a fourth embodiment.
[Fig. 4B] Fig. 4B is a schematic diagram of the subject information obtaining apparatus according to the fourth embodiment.
[Fig. 4C] Fig. 4C is a schematic diagram of the subject information obtaining apparatus according to the fourth embodiment.
[Fig. 5A] Fig. 5A is a schematic diagram of another subject information obtaining apparatus according to the fourth embodiment.
[Fig. 5B] Fig. 5B is a schematic diagram of the other subject information obtaining apparatus according to the fourth embodiment.
[Fig. 5C] Fig. 5C is a schematic diagram of the other subject information obtaining apparatus according to the fourth embodiment.

Description of Embodiments

In photoacoustic imaging, a detection signal obtained by detecting a photoacoustic wave includes background noise. Therefore, in the photoacoustic imaging, it is desirable that initial sound pressure in a region of interest is obtained without using a detection signal that includes background noise and whose S/N is low. For example, in PTL 2, it is described that, although this is a case of ultrasonic imaging, an acoustic wave detection element prevents reception of an acoustic wave from a region of interest when an angle between the region of interest and the acoustic wave detection element is smaller than or equal to a certain value (when the region of interest is not included in a certain sensitive region corresponding to the acoustic wave detection element). By using such a method, an ultrasonic wave image is obtained without using a detection signal whose S/N is low.

Therefore, the present inventor has applied the technology described in PTL 2 to the photoacoustic imaging. More specifically, the initial sound pressure in the region of interest is obtained by performing reconfiguration through simulation without using a detection signal obtained by an acoustic wave detection element whose certain sensitive region does not include the region of interest. Since the initial sound pressure obtained in such a manner is initial sound pressure reconfigured without using a detection signal whose S/N is low, an error due to noise is small. Next, the present inventor has obtained an absorption coefficient in the region of interest using this initial sound pressure and the method described in PTL 1. However, the value of the absorption coefficient calculated using the above method is different from a value of the absorption coefficient set in the simulation.

Therefore, as a result of the present inventor's sincere examination in view of the above problem, it has been found out that the cause of the problem is that whereas detection signals to be used to obtain the initial sound pressure are selected on the basis of the sensitivities of acoustic wave detection elements, the sensitivities of the acoustic wave detection elements are not considered when a value of the integrated light intensity is to be obtained.

Therefore, the present inventor has found out that when the absorption coefficient is to be obtained, the absorption coefficient as an optical characteristic value can be accurately obtained by obtaining a value of the integrated light intensity on the basis of the sensitivities of the acoustic wave detection elements, in addition to selecting detection signals to be used on the basis of the sensitivities of the acoustic wave detection elements.

Embodiments of the present invention using simulation will be described hereinafter.

(First Embodiment)

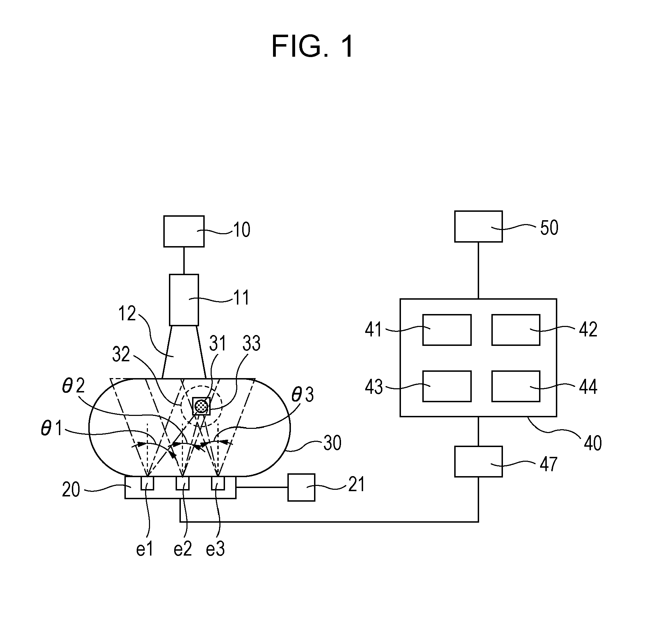

Fig. 1 is a schematic diagram of a subject information obtaining apparatus according to the present embodiment. Pulse light emitted from a light source 10 is guided to an optical system 11 and radiated onto a subject 30 as radiation light 12. A photoacoustic wave 32 generated from a light absorber 31 inside the subject 30 is detected by an acoustic wave detector 20 including acoustic wave detection elements e1, e2, and e3. A plurality of detection signals obtained by the acoustic wave detector 20 are amplified and subjected to digital conversion by a signal collector 47 and stored in a memory of a signal processing apparatus 40. Next, an initial sound pressure obtaining module 42 as an initial sound pressure obtaining unit included in the signal processing apparatus 40 as a signal processing unit reconfigures an image using the plurality of detection signals to obtain initial sound pressure in a region of interest 33 inside the subject 30. In addition, a light intensity obtaining module 43 as a light intensity obtaining unit included in the signal processing apparatus 40 obtains a value of the integrated light intensity in the region of interest 33. Next, an optical characteristic value obtaining module 44 as an optical characteristic value obtaining unit included in the signal processing apparatus 40 obtains an optical characteristic value in the region of interest 33 using the initial sound pressure and the value of the light intensity in the region of interest 33. The obtained optical characteristic value is then displayed on a display apparatus 50 as a display unit.

Here, the region of interest refers to a voxel, which is a minimum unit of a region reconfigured by the initial sound pressure obtaining module 42. It is to be noted that the initial sound pressure obtaining module 42 can obtain the distribution of initial sound pressure of the entirety of the subject by setting the region of interest all over the subject 30. In addition, similarly, the light intensity obtaining module 43 and the optical characteristic value obtaining module 44 can obtain the distribution of values of the integrated light intensity and the distribution of absorption coefficients of the entirety of the subject by setting the region of interest all over the subject.

Here, the detection signals corresponding to the region of interest 33 obtained by the acoustic wave detection elements e1, e2, and e3 illustrated in Fig. 1 are denoted by $P_{d1}(r_T)$, $P_{d2}(r_T)$, and $P_{d3}(r_T)$, respectively. In addition, in relation to a photoacoustic wave that is incident from a front of a photoacoustic wave detection element, the efficiency of conversion from a photoacoustic wave that is incident from an angle $\theta$ relative to the front of the acoustic wave detection element into a detection signal is denoted by $A(\theta)$. In addition, if the angles of the acoustic wave detection elements relative to the region of interest 33 are denoted by $\theta1$, $\theta2$, and $\theta3$, respectively, conversion efficiencies according to the directivity of the acoustic wave detection elements can be expressed as $A(\theta1)$, $A(\theta2)$, and $A(\theta3)$, respectively. In addition, values of the light intensity in the region of interest 33 corresponding to the detection signals $P_{d1}(r_T)$, $P_{d2}(r_T)$, and $P_{d3}(r_T)$ are denoted by $\Phi_1(r_T)$, $\Phi_2(r_T)$, and $\Phi_3(r_T)$, respectively. Here, in the present embodiment, the region of interest 33 is set at a position $r_T$ of the light absorber 31.

Here, the distance from an acoustic wave detection element to the region of interest 33 is denoted by r, the transmission speed of the photoacoustic wave in the subject is denoted by c, and the time at which the radiation light 12 is radiated onto the subject 30 is denoted by t = 0. In this case, the detection signals corresponding to the region of interest refer to detection signals obtained by the acoustic wave detection elements at a time t = r/c. In addition, the values of the light intensity in the region of interest 33 corresponding to the detection signals corresponding to the region of interest refer to values of the intensity of the radiation light 12 in the region of interest 33 radiated at the time t = 0.

(Example of Simulation Not Using Detection Signal)

An example of simulation in which the absorption coefficient is obtained from initial sound pressure obtained without using a detection signal on the basis of the sensitivities of the acoustic wave detection elements will be described hereinafter with reference to Fig. 1. In this simulation, the absorption coefficient of the light absorber 31 was set to $\mu_a =$

0.088/mm.

First, the initial sound pressure obtaining module 42 obtains initial sound pressure $P_0(r_T)$ in the region of interest 33 using the detection signals $P_{d1}(r_T)$, $P_{d2}(r_T)$, and $P_{d3}(r_T)$ and the conversion efficiencies $A(\theta 1)$, $A(\theta 2)$, and $A(\theta 3)$ as represented by an expression (2).

[Math. 2]

$$P_0(r_T) = \frac{P_{d1}(r_T)}{A(\theta 1)} + \frac{P_{d2}(r_T)}{A(\theta 2)} + \frac{P_{d3}(r_T)}{A(\theta 3)} \quad \dots \texttt{Expression (2)}$$

Here, the detection signals obtained by the simulation and the conversion efficiencies set in the simulation were as follows:

$P_{d1}(r_T) = 132$ Pa
$P_{d2}(r_T) = 231$ Pa
$P_{d3}(r_T) = 198$ Pa
$A(\theta 1) = 0.4$
$A(\theta 2) = 0.7$
$A(\theta 3) = 0.6$

Accordingly, the initial sound pressure calculated from the expression (2) using these parameters is $P_0(r_T) = 990$.

In addition, in Fig. 1, regions (certain sensitive regions) in which the conversion efficiencies of the acoustic wave detection elements are larger than a certain value are represented by triangular regions indicated by broken lines. Here, a conversion efficiency $A(\theta) = 0.5$ is set as the certain value.

Here, in the present embodiment, the region of interest 33 is not included in the triangular region (the certain sensitive region) corresponding to the acoustic wave detection element e1. Therefore, the initial sound pressure obtaining module 42 obtains initial sound pressure $P_0'(r_T)$ in the region of interest 33 represented by an expression (3) without using the detection signal $P_{d1}(r_T)$ corresponding to the region of interest 33 obtained by the acoustic wave detection element e1.

[Math. 3]

$$P_0'(r_T) = \frac{P_{d2}(r_T)}{A(\theta 2)} + \frac{P_{d3}(r_T)}{A(\theta 3)} \quad \dots \texttt{Expression (3)}$$

Accordingly, the initial sound pressure in the region of interest 33 calculated from the expression (3) using the above parameters was $P_0'(r_T) = 660$.

Next, the light intensity obtaining module 43 obtains a value of the integrated light intensity in the subject from a background optical coefficient of the subject or the like using a light propagation Monte Carlo method, a transport equation, a light diffusion equation, or the like.

For example, the light intensity obtaining module 43 obtains the values of the light intensity $\Phi_1(r_T)$, $\Phi_2(r_T)$, and $\Phi_3(r_T)$ in the region of interest 33 corresponding to the detection signals $P_{d1}(r_T)$, $P_{d2}(r_T)$, and $P_{d3}(r_T)$, respectively.

The light intensity obtaining module 43 then obtains the value of the integrated light intensity $\Phi(r_T)$ in the region of interest 33 represented by an expression (4) using these values.

[Math. 4]

$$\Phi(r_T) = \Phi_1(r_T) + \Phi_2(r_T) + \Phi_3(r_T) \quad \dots \texttt{Expression (4)}$$

Here, the values of the light intensity in the region of interest obtained by the simulation were as follows:

$\Phi_1(r_T) = 3,750$ mJ/m$^2$
$\Phi_2(r_T) = 3,750$ mJ/m$^2$
$\Phi_3(r_T) = 3,750$ mJ/m$^2$

Accordingly, the value of the integrated light intensity in the region of interest from the expression (4) using these parameters is $\Phi(r_T)$ = 11,250 mJ/m$^2$.

Next, the optical characteristic value obtaining module 44 obtains the absorption coefficient $\mu_a(r_T)$ in the region of interest 33 represented by an expression (5) using the initial sound pressure $P_0'(r_T)$ in the region of interest 33 represented by the expression (3) and the value of the integrated light intensity $\Phi(r_T)$ in the region of interest 33 represented by the expression (4).

Here, a Grueneisen coefficient $\Gamma$ = 1.

[Math. 5]

$$\mu_a(r_T) = \frac{\dfrac{P_{d2}(r_T)}{A(\theta 2)} + \dfrac{P_{d3}(r_T)}{A(\theta 3)}}{\Phi_1(r_T) + \Phi_2(r_T) + \Phi_3(r_T)} \quad \text{... Expression (5)}$$

Here, the absorption coefficient in the region of interest 33 set at the position $r_T$ of the light absorber, the absorption coefficient being calculated by the expression (5) using the above parameters, is $\mu_a$ = 0.059/mm. On the other hand, the absorption coefficient of the light absorber 31 set in the simulation is $\mu_a$ = 0.088/mm. Therefore, it can be seen that the absorption coefficient obtained by the expression (5) is smaller than the set value. That is, when the absorption coefficient is to be obtained using the initial sound pressured obtained using the above method, a further improvement is needed to obtain the value of the integrated light intensity.

(Example of Simulation Not Using Detection Signal And Value of Light Intensity)

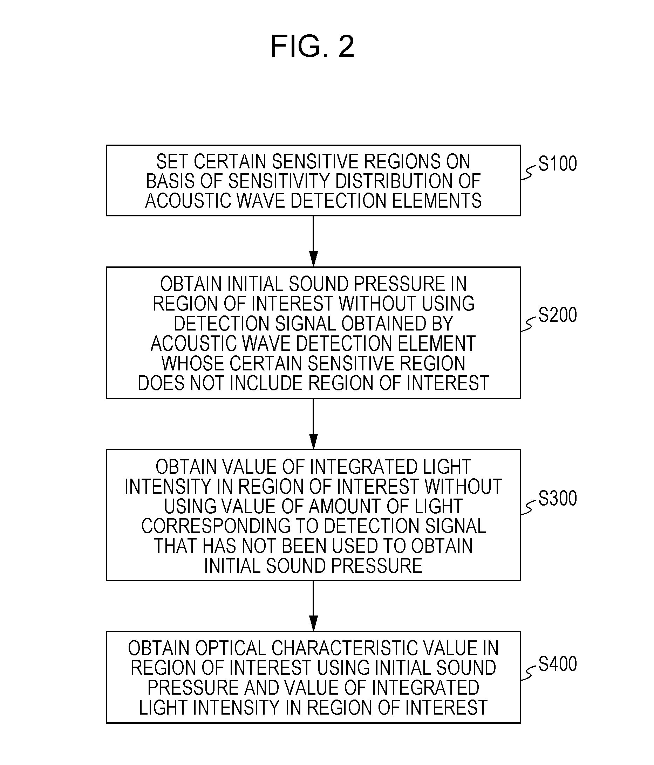

Therefore, a method for obtaining information regarding a subject according to the present embodiment found by the present inventor will be described hereinafter with reference to a flowchart of Fig. 2. The following numbers agree with processing numbers illustrated in Fig. 2.

(S100: Step of Setting Certain Sensitive Regions on Basis of Sensitivity Distribution of Acoustic Wave Detection Elements)

In this step, certain sensitive regions corresponding to the plurality of acoustic wave detection elements are set on the basis of the sensitivity distribution of the plurality of acoustic wave detection elements. Tables of the certain sensitive regions corresponding to the acoustic wave detection elements are stored in the memory of the signal processing apparatus 40.

Here, a setting module 41 as a setting unit included in the signal processing apparatus 40 may set regions in which the sensitivities of the acoustic wave detection elements are higher than a certain value as the certain sensitive regions. It is to be noted that the certain value may be automatically set by the setting module 41 on the basis of system noise. In addition, the certain value may be determined by an operator by displaying the sensitivities of the acoustic wave detection elements on the display apparatus 50 as a histogram and by selecting the certain value on the basis of the histogram. At this time, the certain value is preferably selected in consideration of the system noise.

Here, the sensitivities of the acoustic wave detection elements are determined from, for example, the conversion efficiencies of the acoustic wave detection elements, attenuation rates, which indicate attenuation caused by diffusion and scattering of the photoacoustic wave from the region of interest to the acoustic wave detection elements, and the like. It is to be noted that the conversion efficiencies are determined from angles at which the photoacoustic wave is incident on the acoustic wave detection elements and the like. In addition, the attenuation rates are determined from the distances between the region of interest and the acoustic wave detection elements and the like.

For example, in the case of the example of simulation described above, the setting module 41 set the conversion efficiency $A(\theta)$ = 0.5 as the certain value. In addition, with respect to each of the acoustic wave detection elements e1, e2, and e3, a region in which the conversion efficiency $A(\theta)$ is higher than 0.5 was indicated by a triangular region. As a result, the region of interest 33 was not included in the region (the certain sensitive region) in which the conversion efficiency $A(\theta)$ of the acoustic wave detection element e1 is higher than 0.5.

In addition, the certain sensitive regions can be set on the basis of arbitrary regions selected in an image of the sensitivity distribution of the acoustic wave detection elements.

For example, first, the display apparatus 50 is caused to display image data regarding the sensitivity distribution of the acoustic wave detection elements stored in the memory of the signal processing apparatus 40. The operator then selects arbitrary regions using an input device of a PC in the displayed image of the sensitivity distribution. Therefore, the setting module 41 can set the selected arbitrary regions as the certain sensitive regions. At this time, for example, each arbitrary region can be selected by connecting a start point and an end point using recognition by a mouse or a recognition method by a sensor on a touch panel while the image of the sensitive region is being displayed.

It is to be noted that the setting module 41 may set the certain sensitive regions on the basis of the sensitivity distribution of the selected arbitrary regions, instead. For example, the certain sensitive regions may be set using the lowest sensitivity as a reference.

In addition, a certain sensitive region may be individually set for each of the acoustic wave detection elements or a certain sensitive region may be set for a single acoustic wave detection element and the same sensitive region as the certain sensitive region may be set for each of the other acoustic wave detection elements.

(S200: Step of Obtaining Initial Sound Pressure in Region of Interest without Using Detection Signal Obtained by Acoustic Wave Detection Element Whose Certain Sensitive Region Does Not Include Region of Interest)

In this step, the initial sound pressure in the region of interest is obtained without using a detection signal corresponding to the region of interest obtained by an acoustic wave detection element whose certain sensitive region set in S100 does not include the region of interest. Thereafter, data regarding the initial sound pressure is stored in the memory of the signal processing apparatus 40.

For example, in the case of the example of simulation described above, the region of interest 33 was not included in the certain sensitive region corresponding to the acoustic wave detection element e1. Therefore, the initial sound pressure obtaining module 42 obtains the initial sound pressure $P_0'(r_T)$ represented by the expression (4) by reconfiguring an image without using, among the detection signals $P_{d1}(r_T)$, $P_{d2}(r_T)$, and $P_{d3}(r_T)$, the detection signal $P_{d1}(r_T)$ corresponding to the region of interest obtained by the acoustic wave detection element e1.

At this time, as an image reconfiguration algorithm executed by the initial sound pressure obtaining module 42, for example, reverse projection in a time domain or a Fourier domain normally used in a tomography technology or the like may be used.

It is to be noted that, in the present invention, if a certain sensitive region is included in at least a part of the region of interest, it can be said that the region of interest is included in the certain sensitive region.

In addition, in the present invention, not using a detection signal is a concept including not using a detection signal at all and not using a detection signal essentially when the initial sound pressure is obtained.

(S300: Step of Obtaining Value of Integrated Light Intensity in Region of Interest without Using Value of Light Intensity Corresponding To Detection Signal Not Used To Obtain Initial Sound Pressure)

In this step, the value of the integrated light intensity in the region of interest is obtained without using the value of the light intensity in the region of interest corresponding to the detection signal that has not been used in S200. Thereafter, data regarding the value of the integrated light intensity is stored in the memory of the signal processing apparatus 40.

For example, the light intensity obtaining module 43 obtains a value of the integrated light intensity $\Phi'(r_T)$ in the region of interest represented by an expression (6) without using, among the values of the light intensity $\Phi_1(r_T)$, $\Phi_2(r_T)$, and $\Phi_3(r_T)$, the value of the light intensity $\Phi_1(r_T)$ in the region of interest corresponding to the detection signal $P_{d1}(r_T)$ that has not been used by the initial sound pressure obtaining module 42.

[Math. 6]

$$\Phi'(r_T) = \Phi_2(r_T) + \Phi_3(r_T) \ \text{... Expression (6)}$$

That is, the light intensity obtaining module 43 obtains the value of the integrated light intensity in the region of interest using the values of the light intensity in the region of interest corresponding to detection signals used when the initial sound pressure obtaining module 42 has calculated the initial sound pressure.

Here, the value of the integrated light intensity in the region of interest 33 obtained by the expression (6) using the above parameters is $\Phi'(r_T) = 7,500 \ \text{mJ/m}^2$.

It is to be noted that, in the present invention, not using a value of the light intensity is a concept including not using a value of the light intensity at all and not using a value of the light intensity essentially when the value of the integrated light intensity is obtained.

In addition, in the present embodiment, since radiation conditions of the radiation light 12 are constant, the light intensity radiated onto the region of interest 33 remains the same. Therefore, the values of the light intensity corresponding to the plurality of detection signals obtained by the plurality of acoustic wave detection elements also remain the same. In such a case, the light intensity obtaining module 43 may obtain a value obtained by multiplying the number of detection signals used when the initial sound pressure obtaining module 42 has obtained the initial sound pressure by the light intensity that has reached the region of interest 33 as the value of the integrated light intensity in the region of interest 33. In the present invention, the value of the integrated light intensity obtained in such a manner is treated as the value of the integrated light intensity obtained without using a value of the light intensity.

(S400: Step of Obtaining Optical Characteristic Value in Region of Interest Using Initial Sound Pressure And Value of Integrated Light Intensity in Region of Interest)

In this step, the absorption coefficient as an optical characteristic value in the region of interest is obtained using the initial sound pressure in the region of interest obtained in S200 and the value of the integrated light intensity in the region of interest obtained in S300.

For example, in the case of the example of simulation described above, the optical characteristic value obtaining module 44 applies the initial sound pressure $P_0'(r_T)$ represented by the expression (3) and the value of the integrated light intensity $\Phi'(r_T)$ represented by the expression (6) to the expression (1). In doing so, an absorption coefficient $\mu_a(r_T)$ in the region of interest 33 represented by the expression (7) is obtained. Here, the Grueneisen coefficient $\Gamma = 1$.

[Math. 7]

$$\mu_a(r_T) = \frac{P_0'(r_T)}{\Phi'(r_T)} = \frac{\dfrac{P_{d2}(r_T)}{A(\theta 2)} + \dfrac{P_{d3}(r_T)}{A(\theta 3)}}{\Phi_2(r_T) + \Phi_3(r_T)} \quad \dots \text{Expression (7)}$$

For example, the absorption coefficient in the region of interest 33 obtained by the expression (7) using the above parameters is $\mu_a = 0.088$/mm. On the other hand, the absorption coefficient in the region of interest 33 obtained by the expression (5) is $\mu_a = 0.059$/mm. In addition, the absorption coefficient of the light absorber 31 set in the simulation is $\mu_a = 0.088$/mm. That is, according to the expression (7), the absorption coefficient can be obtained accurately compared to the expression (5).

As described above, an absorption coefficient in which an error due to noise is small and whose quantativity is high can be obtained by, with respect to an acoustic wave detection element whose certain sensitive region does not include the region of interest, not using a detection signal corresponding to the region of interest obtained by the acoustic wave detection element and a value of the light intensity in the region of interest corresponding to the detection signal.

It is to be noted that by using the above steps for a plurality of wavelengths, an absorption coefficient for each wavelength may be obtained, instead. In addition, using these absorption coefficients, oxygen saturation as an optical characteristic value may be obtained.

Alternatively, a program including the above steps may be executed by the signal processing apparatus 40 as a computer.

(Second Embodiment)

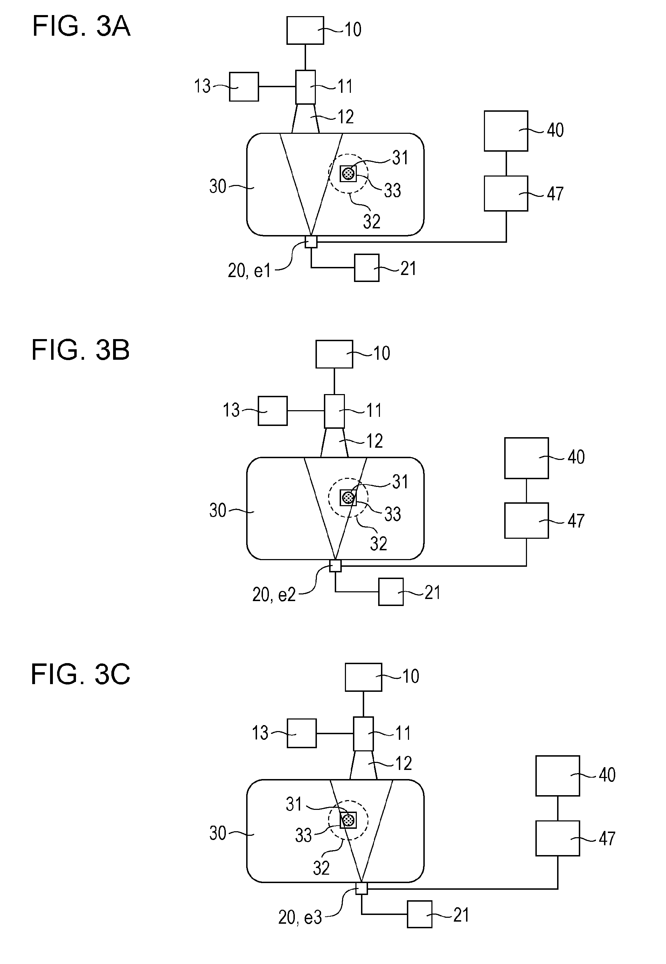

Figs. 3A to 3C are schematic diagrams of a subject information obtaining apparatus according to the present embodiment.

The subject information obtaining apparatus according to the present embodiment includes an acoustic wave detector 20 including a single acoustic wave detection element. In addition, a detector moving mechanism 21 for moving the subject 30 and the acoustic wave detector 20 relatively to each other is included. In the present embodiment, the detector moving mechanism 21 can detect the photoacoustic wave at a plurality of positions by moving the acoustic wave detector 20 including the single acoustic wave detection element in a right direction of the paper. Here, the acoustic wave detection element at positions illustrated in Figs. 3A, 3B, and 3C is denoted by e1, e2, and e3, respectively. In addition, triangular regions indicated by lines represent a certain sensitive region corresponding to the acoustic wave detection element.

In the present invention, a plurality of acoustic wave detection elements refer to an acoustic wave detection element that can detect the photoacoustic wave at a plurality of positions. That is, as in the present embodiment, an acoustic wave detection element that can detect the photoacoustic wave at a plurality of positions by moving the acoustic wave detector 20 is also referred to as a plurality of acoustic wave detection elements.

In addition, the subject information obtaining apparatus according to the present embodiment is provided with an optical moving mechanism 13 that moves the optical system 11 in order to move the radiation light 12. In addition, in the present embodiment, the acoustic wave detector 20 and the radiation light 12 are moved in synchronization with each other. Thus, by moving the acoustic wave detector 20 and the radiation light 12 in synchronization with each other, the radiation light 12 is constantly radiated onto the certain sensitive region (triangular region) corresponding to the acoustic wave detection element, thereby making it possible to constantly obtain a detection signal whose S/N is high.

In the subject information obtaining apparatus according to the present embodiment, too, as in the first embodiment, the region of interest 33 is not included in the certain sensitive region corresponding to the acoustic wave detection element e1. Therefore, the initial sound pressure obtaining module 42 obtains the initial sound pressure in the region of interest 33 without using a detection signal corresponding to the region of interest 33 obtained by the acoustic wave detection element e1. Thereafter, the light intensity obtaining module 43 obtains the value of the integrated light intensity in the region of interest 33 without using a value of the light intensity in the region of interest 33 corresponding to the detection signal that has not been used to obtain the initial sound pressure. Thereafter, the optical characteristic value obtaining module 44 obtains the absorption coefficient in the region of interest 33 represented by the expression (7) using the initial sound pressure and the value of the integrated light intensity. By obtaining the absorption coefficient in such a manner, in the present embodiment, too, the absorption coefficient can be accurately obtained.

(Third Embodiment)

In the first embodiment and the second embodiment, the absorption coefficient is obtained without using, with respect to an acoustic wave detection element whose certain sensitive region does not include the region of interest, a detection signal corresponding to the region of interest obtained by the acoustic wave detection element and a value of the light intensity in the region of interest corresponding to the detection signal. On the other hand, in the present embodiment, the absorption coefficient is obtained while reducing the detection signal and the value of the light intensity in the region of interest corresponding to the detection signal.

A method for obtaining information regarding a subject according to the present embodiment will be described hereinafter using the subject information obtaining apparatus illustrated in Fig. 1.

In the present embodiment, the initial sound pressure obtaining module 42 multiplies the detection signal corresponding to the region of interest 33 obtained by the acoustic wave detection element e1 whose certain sensitive region does not include the region of interest 33 by a first reduction coefficient. The initial sound pressure obtaining module 42 then obtains the initial sound pressure in the region of interest 33 using the detection signal multiplied by the first reduction coefficient, too.

Thus, by multiplying, with respect to an acoustic wave detection element whose certain sensitive region does not include the region of interest, a detection signal corresponding to the region of interest obtained by the acoustic wave detection element by the first reduction coefficient, the initial sound pressure can be obtained while reducing the detection signal, whose S/N is low. Therefore, initial sound pressure in which an error due to noise is small can be obtained.

Next, the light intensity obtaining module 43 multiplies, by a second reduction coefficient, the value of the light intensity in the region of interest 33 corresponding to the detection signal multiplied by the first reduction coefficient. The light intensity obtaining module 43 then obtains the value of the integrated light intensity in the region of interest 33 using the value of the light intensity multiplied by the second reduction coefficient, too.

Thereafter, the optical characteristic value obtaining module 44 obtains the absorption coefficient in the region of interest 33 using the initial sound pressure obtained by the initial sound pressure obtaining module 42 and the value of the integrated light intensity obtained by the light intensity obtaining module 43.

Thus, by multiplying the value of the light intensity corresponding to the detection signal by the second reduction coefficient in addition to multiplying the detection signal by the first reduction coefficient, the absorption coefficient can be accurately obtained.

It is to be noted that the first reduction coefficient and the second reduction coefficient are values smaller than 1. Alternatively, another reduction coefficient may be set in accordance with the region of interest. In addition, it is preferable that the first reduction coefficient and the second reduction coefficient are the same value. Here, the same value is a concept including the completely same value and values that become essentially the same when the absorption coefficient is obtained.

(Fourth Embodiment)

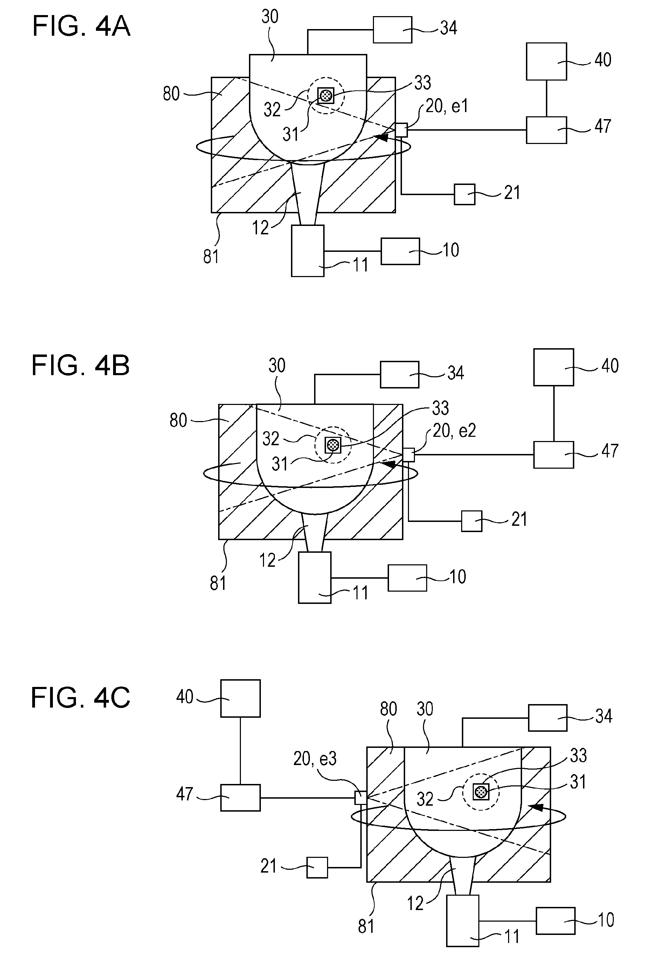

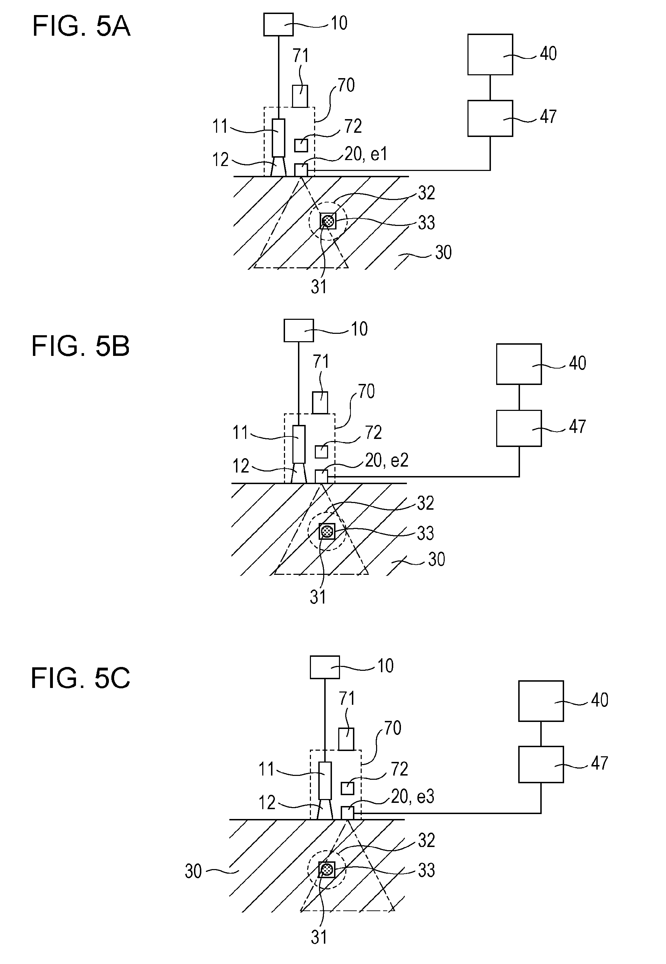

The present invention can be applied to a subject information obtaining apparatus illustrated in Figs. 4A to 4C and a subject information obtaining apparatus illustrated in Figs. 5A to 5C. The subject information obtaining apparatus illustrated in Figs. 4A to 4C can detect the photoacoustic wave at a plurality of positions by rotatively moving the acoustic wave detector 20 around the subject 30 using the detector moving mechanism 21. In addition, in order to establish acoustic impedance matching between the subject 30 and the acoustic wave detector 20, the subject 30 is immersed in water 80 that fills a water tank 81. In addition, a subject moving mechanism 34 that moves the subject 30 is included. By using such a configuration, a portion whose shape cannot be defined by a holding board or the like can be measured. In addition, because detection elements can be set in a lot of directions relative to the subject, data whose amount of information is large can be obtained.

In the case of the subject information obtaining apparatus illustrated in Figs. 4A to 4C, a state illustrated in Fig. 4A changes to a state illustrated in Fig. 4B by moving the subject in a lower direction of the paper using the subject moving mechanism 34. In addition, the state illustrated in Fig. 4B changes to a state illustrated in Fig. 4C by moving the acoustic wave detector 20 using the detector moving mechanism 21. Here, the acoustic wave detection element in the states illustrated in Figs. 4A, 4B, and 4C is denoted by e1, e2, and e3, respectively. In addition, triangular regions indicated by broken lines represent a certain sensitive region corresponding to the acoustic wave detection element.

In addition, the subject information obtaining apparatus illustrated in Figs. 5A to 5C is provided with the acoustic wave detector 20 and the optical system 11 stored in a single housing 70. In addition, the housing 70 is provided with a hand-held mechanism 71, and the operator can move the housing 70 by grasping the hand-held mechanism 71. By moving the housing 70 in such a manner, the acoustic wave detection element can detect the photoacoustic wave at a plurality of positions. In Figs. 5A to 5C, the acoustic wave detection element detects the photoacoustic wave while the operator is moving the housing 70 in a right direction of the paper by grasping the hand-held mechanism 71. Here, the acoustic wave detection element in states illustrated in Figs. 5A, 5B, and 5C is denoted by e1, e2, and e3, respectively. In addition, triangular regions indicated by broken lines represent a certain sensitive region corresponding to the acoustic wave detection element.

However, in the present embodiment, unlike the other embodiments, the acoustic wave detector 20 is moved not mechanically but the housing 70 is arbitrarily moved by the operator by grasping the hand-held mechanism 71. Therefore, the positional relationship between the acoustic wave detector 20 and the region of interest 33 when the photoacoustic wave 32 has been detected cannot be identified. However, in order to extract a detection signal corresponding to the region of interest from a detection signal obtained by the acoustic wave detector 20, it is necessary to identify the positional relationship between the acoustic wave detector 20 and the region of interest 33. Therefore, in the present embodiment, it is preferable that the housing 70 includes a position detector 72 for detecting the position of the housing 70, that is, the positions of the acoustic wave detector 20 and the optical system 11 stored in the housing 70.

In the cases of the subject information obtaining apparatuses illustrated in Figs. 4A to 4C and Figs. 5A to 5C, the certain sensitive region corresponding to the acoustic wave detection element e1 does not include the region of interest 33. Therefore, the signal processing apparatus 40 can obtain the absorption coefficient in the region of interest 33 using the method for obtaining information regarding a subject described in the first and second embodiments or the method for obtaining information regarding a subject described in the third embodiment. By obtaining the absorption coefficient in such a manner, in the present embodiment, too, the absorption coefficient can be accurately obtained.

A principal configuration will be described hereinafter.

(Light Source 10)

The light source 10 includes a light source that can generate pulse light of 5 nanoseconds to 50 nanoseconds. As the light source, a laser from which a large output can be obtained is preferable, but a light-emitting diode may be used instead of the laser. As the laser, one of various lasers such as a solid-state laser, a gas laser, a dye laser, and a semiconductor laser may be used. Ideally, a Ti:Sa laser excited by Nd:YAG or an alexandrite laser that has a large output and whose wavelength can be continuously changed is used. A plurality of single-wavelength lasers having different wavelengths may be included, instead.

(Optical System 11)

The pulse light emitted from the light source 10 is guided to the subject while being processed in such a way as to have a desired optical distribution shape typically by optical components such as lenses and mirrors, but it is also possible to transmit the pulse light using an optical waveguide such as an optical fiber. The optical system 11 is, for example, a mirror that reflects light, a lens that collects or spreads light or changes the shape of light, a diffusion plate that diffuses light, and the like. Such optical components may be any components insofar as the pulse light emitted from the light source is radiated onto the subject in a desired shape. It is to be noted that it is preferable that the light is spread to a certain area compared to that the light is collected by a lens, in terms of the security of the subject and an increase in a diagnostic range. It is to be noted that an optical moving mechanism may be provided in the optical system 11 in order to move the radiation light.

(Acoustic Wave Detector 20)

The acoustic wave detector 20, which is a detector that detects a photoacoustic wave generated on a surface of and an inside of the subject using light, detects an acoustic wave and converts the acoustic wave into an electrical signal that is an analog signal. The acoustic wave detector 20 will also be referred to simply as a probe or a transducer hereinafter. Any type of acoustic wave detector such as a transducer using a piezoelectric phenomenon, a transducer using optical resonance, or a transducer using changes in capacitance may be used insofar as the transducer can detect photoacoustic wave signals.

In addition, the acoustic wave detector 20 includes a plurality of acoustic wave detection elements. By arranging the plurality of acoustic wave detection elements in one dimension or two dimensions as an array, a photoacoustic wave can be detected at a plurality of positions. By using such multidimensionally arranged elements, an acoustic wave can be simultaneously detected at a plurality of positions, thereby reducing detection time and an effect such as vibration of the subject.

It is to be noted that, in order to enable detection of the photoacoustic wave at a plurality of positions, the acoustic wave detector 20 may be configured such that the acoustic wave detector 20 can be mechanically moved by the detector moving mechanism 21. In addition, a hand-held mechanism that is grasped by the operator in order to arbitrarily move the acoustic wave detector 20 may also be included.

(Signal Collector 47)

It is preferable that the signal collector 47 that amplifies an electrical signal obtained by the acoustic wave detector 20 and that converts the electrical signal from an analog signal to a digital signal is included. The signal collector 47 is typically configured by an amplifier, an A/D convertor, an FPGA (Field Programmable Gate Array) chip, and the like. When a plurality of detection signals are obtained by the acoustic wave detector, it is desirable that the plurality of signals can be simultaneously processed. Accordingly, the time until an image is formed can be reduced. It is to be noted that the "detection signal" herein is a concept including both an analog signal output from the acoustic wave detector 20 and a digital signal obtained by AD conversion by the signal collector 47.

(Signal Processing Apparatus 40)

The signal processing apparatus 40 obtains an optical characteristic value inside the subject by performing reconfiguration of an image or the like. As the signal processing apparatus 40, a workstation or the like is typically used, and a process for reconfiguring an image or the like is typically performed by software that has been programmed in advance. For example, the software used in the workstation includes the setting module 41, the initial sound pressure obtaining module 42, the light intensity obtaining module 43, the optical characteristic value obtaining module 44, and the like.

It is to be noted that each module may be provided as a separate piece of hardware. In this case, the pieces of hardware as a whole may be used as the signal processing apparatus 40.

In addition, the signal collector 47 and the signal processing apparatus 40 may be integrated with each other depending on the case. In this case, an optical characteristic value of the subject can be generated not by a software process such as one performed in the workstation but by a hardware process.

The present invention is not limited to the above embodiments and can be changed and modified in various ways without deviating from the spirit and the scope thereof. Therefore, in order to make public the scope of the present invention, the following claims are attached. Reference Signs List

20    acoustic wave detector
30    subject
40    signal processing apparatus
41    setting module
42    initial sound pressure obtaining module
43    light intensity obtaining module
44    optical characteristic value obtaining module

**Claims**

1. A subject information obtaining apparatus comprising:

    an acoustic wave detector that includes a plurality of acoustic wave detection elements configured to detect a photoacoustic wave generated by radiating light onto a subject; and
    a signal processing unit configured to obtain an optical characteristic value in a region of interest using a plurality of detection signals corresponding to the region of interest inside the subject obtained by the plurality of acoustic wave detection elements and a value of integrated light intensity in the region of interest,
    wherein the signal processing unit includes
    a setting unit configured to set certain sensitive regions on the basis of sensitivity distribution of the plurality of acoustic wave detection elements,
    an initial sound pressure obtaining unit configured to obtain initial sound pressure in the region of interest without using a detection signal corresponding to the region of interest obtained by an acoustic wave detection element whose certain sensitive region does not include the region of interest,
a light intensity obtaining unit configured to obtain the value of the integrated light intensity in the region of interest without using a value of light intensity in the region of interest corresponding to the detection signal that is not used by the initial sound pressure obtaining unit, and
an optical characteristic value obtaining unit configured to obtain the optical characteristic value in the region of interest using the initial sound pressure obtained by the initial sound pressure obtaining unit and the value of the integrated light intensity obtained by the light intensity obtaining unit.

2. The subject information obtaining apparatus according to Claim 1,
wherein, in the sensitivity distribution of the acoustic wave detection elements, the setting unit is configured to set regions whose sensitivities are larger than a certain value as the certain sensitive regions.

3. The subject information obtaining apparatus according to Claim 1, further comprising:

a display unit configured to display an image of the sensitivity distribution of the acoustic wave detection elements on the basis of the sensitivity distribution of the acoustic wave detection elements,
wherein the setting unit is configured to set regions based on arbitrary regions selected in the image of the sensitivity distribution of the acoustic wave detection elements displayed on the display unit as the certain sensitive regions.

4. The subject information obtaining apparatus according to any of Claims 1 to 3, further comprising:

a moving mechanism configured to move the acoustic wave detector and the subject relatively to each other.

5. The subject information obtaining apparatus according to any of Claims 1 to 4,
wherein the sensitivities of the acoustic wave detection elements are set on the basis of at least either an efficiency of conversion from the photoacoustic wave into the detection signals performed by the acoustic wave detection elements or an attenuation rate of the photoacoustic wave from the region of interest to the acoustic wave detection elements.

6. A subject information obtaining apparatus comprising:

an acoustic wave detector that includes a plurality of acoustic wave detection elements configured to detect a photoacoustic wave generated by radiating light onto a subject; and
a signal processing unit configured to obtain an optical characteristic value in a region of interest using a plurality of detection signals corresponding to the region of interest inside the subject obtained by the plurality of acoustic wave detection elements and a value of an integrated light intensity in the region of interest,
wherein the signal processing unit includes
a setting unit configured to set certain sensitive regions on the basis of sensitivity distribution of the plurality of acoustic wave detection elements,
an initial sound pressure obtaining unit configured to obtain initial sound pressure in the region of interest by multiplying a detection signal corresponding to the region of interest obtained by an acoustic wave detection element whose certain sensitive region does not include the region of interest by a first reduction coefficient,
a light intensity obtaining unit configured to obtain the value of the integrated light intensity in the region of interest by multiplying, by a second reduction coefficient, a value of light intensity in the region of interest corresponding to the detection signal multiplied by the first reduction coefficient by the initial sound pressure obtaining unit, and
an optical characteristic value obtaining unit configured to obtain the optical characteristic value in the region of interest using the initial sound pressure obtained by the initial sound pressure obtaining unit and the value of the integrated light intensity obtained by the light intensity obtaining unit.

7. The subject information obtaining apparatus according to Claim 6,
wherein the first reduction coefficient and the second reduction coefficient are the same value.

8. A method for obtaining information regarding a subject, the method comprising the steps of:

detecting, by a plurality of acoustic wave detection elements, a photoacoustic wave generated by radiating light onto the subject in order to obtain a plurality of detection signals in a region of interest inside the subject;

obtaining an optical characteristic value in the region of interest using the plurality of detection signals and a value of integrated light intensity in the region of interest;

setting certain sensitive regions on the basis of sensitivity distribution of the plurality of acoustic wave detection elements;

obtaining initial sound pressure in the region of interest without using a detection signal corresponding to the region of interest obtained by an acoustic wave detection element whose certain sensitive region does not include the region of interest;

obtaining the value of the integrated light intensity in the region of interest without using a value of light intensity in the region of interest corresponding to the detection signal that is not used in the step of obtaining the initial sound pressure; and

obtaining the optical characteristic value in the region of interest using the initial sound pressure obtained in the step of obtaining the initial sound pressure and the value of the integrated light intensity obtained in the step of obtaining the value of the integrated light intensity.

9. A method for obtaining information regarding a subject, the method comprising the steps of:

detecting, by a plurality of acoustic wave detection elements, a photoacoustic wave generated by radiating light onto the subject in order to obtain a plurality of detection signals in a region of interest inside the subject;

obtaining an optical characteristic value in the region of interest using the plurality of detection signals and a value of integrated light intensity in the region of interest;

setting certain sensitive regions on the basis of sensitivity distribution of the plurality of acoustic wave detection elements;

obtaining initial sound pressure in the region of interest by multiplying a detection signal corresponding to the region of interest obtained by an acoustic wave detection element whose certain sensitive region does not include the region of interest by a first reduction coefficient;

obtaining the value of the integrated light intensity in the region of interest by multiplying, by a second reduction coefficient, a value of light intensity in the region of interest corresponding to the detection signal multiplied by the first reduction coefficient in the step of obtaining the initial sound pressure; and

obtaining the optical characteristic value in the region of interest using the initial sound pressure obtained in the step of obtaining the initial sound pressure and the value of the integrated light intensity obtained in the step of obtaining the value of the integrated light intensity.

10. A program for causing a computer to execute the method for obtaining information regarding a subject according to Claim 8 or 9.

# FIG. 1

# FIG. 2

SET CERTAIN SENSITIVE REGIONS ON
BASIS OF SENSITIVITY DISTRIBUTION OF
ACOUSTIC WAVE DETECTION ELEMENTS — S100

↓

OBTAIN INITIAL SOUND PRESSURE IN
REGION OF INTEREST WITHOUT USING
DETECTION SIGNAL OBTAINED BY
ACOUSTIC WAVE DETECTION ELEMENT
WHOSE CERTAIN SENSITIVE REGION
DOES NOT INCLUDE REGION OF INTEREST — S200

↓

OBTAIN VALUE OF INTEGRATED LIGHT
INTENSITY IN REGION OF INTEREST WITHOUT
USING VALUE OF AMOUNT OF LIGHT
CORRESPONDING TO DETECTION SIGNAL
THAT HAS NOT BEEN USED TO OBTAIN
INITIAL SOUND PRESSURE — S300

↓

OBTAIN OPTICAL CHARACTERISTIC VALUE IN
REGION OF INTEREST USING INITIAL SOUND
PRESSURE AND VALUE OF INTEGRATED
LIGHT INTENSITY IN REGION OF INTEREST — S400

**FIG. 3A**

**FIG. 3B**

**FIG. 3C**

FIG. 4A

FIG. 4B

FIG. 4C

FIG. 5A

FIG. 5B

FIG. 5C

| | INTERNATIONAL SEARCH REPORT | International application No. |
| --- | --- | --- |
| | | PCT/JP2012/050914 |

**A. CLASSIFICATION OF SUBJECT MATTER**
*A61B8/00*(2006.01)i

According to International Patent Classification (IPC) or to both national classification and IPC

**B. FIELDS SEARCHED**

Minimum documentation searched (classification system followed by classification symbols)
A61B8/00

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)
WPI, PubMed, CiNii, JMEDPlus(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2011-245277 A (Canon Inc.), 08 December 2011 (08.12.2011), entire text; all drawings (Family: none) | 1-10 |
| A | JP 2006-51355 A (General Electric Co.), 23 February 2006 (23.02.2006), entire text; all drawings & US 2006/0058670 A1 & DE 102005037823 A & CN 1734286 A | 1-10 |

☐ Further documents are listed in the continuation of Box C.  ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 29 February, 2012 (29.02.12) | 13 March, 2012 (13.03.12) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010088627 A
- JP 2006051355 A